# FASCICULE DE BREVET EUROPEEN

(11) **EP 0 621 014 B1**
(45) Date de publication et mention de la délivrance du brevet: **16.08.2000**
(21) Numéro de dépôt: 94420114.4
(22) Date de dépôt: 08.04.1994
(51) Int. Cl.: A61F 2/00

(54) **Ensemble prothétique en matériau textile**
Prosthetische Vorrichtung aus Textilmaterial
Prosthetic device made of fabric material

(30) Priorité: 23.04.1993 FR 9305185
(43) Date de publication de la demande: 26.10.1994
(73) Titulaire: Sgro, Jean-Claude, F-21000 Dijon (FR)
(72) Inventeur: Sgro, Jean-Claude, F-21000 Dijon (FR)
(74) Mandataire: Hubert, Philippe

(56) Documents cités:
- WO-A-89/08467
- WO-A-92/19162
- DE-C- 907 115
- GB-A- 1 062 876
- US-A- 3 124 136
- US-A- 4 769 038
- US-A- 4 854 316
- US-A- 5 147 374

## Description

L'invention se rattache au secteur technique des prothèses vasculaires implantables chez l'être humain ainsi qu'aux prothèses de renfort ou de remplacement de la paroi abdominale et plus généralement des pertes de substances musculaires et aponévrotiques.

Plus particulièrement, l'invention concerne une prothèse du type de celle décrite dans le brevet FR 2541888 dont le demandeur de la présente est également titulaire. Pour l'essentiel, ce brevet divulgue une prothèse partiellement résorbable comprenant un support interne en matière synthétique souple non résorbable, un revêtement résorbable en polyglycol ou collagène coopérant avec le support interne et enfin, un élément de renfort externe non résorbable tissé ou tricoté, à mailles plus larges. Les trois éléments peuvent être disposés en couches concentriques, superposées ou imbriquées.

Les parties résorbables permettent le passage des cellules des tissus avoisinants destinés à tapisser la prothèse. Au contact des cellules, les éléments du sang destinés à la coagulation ne sont pas activés, ce qui permet une diminution des risques d'oblitération. Les parties non résorbables de la prothèse garantissent la solidité et la souplesse.

On connaît également des prothèses du type de celles décrites dans le brevet WO 92/19162. L'élément prothétique se présente sous forme d'un champignon présentant une partie centrale de forme cylindrique accouplée à deux plaques planes par exemple par un système du type de ceux connus sous la marque VELCRO. Cet élément prothétique constitue un bouchon qui empêche la cicatrisation. Un tel élément est difficilement applicable pour le traitement d'hernies inguinales et d'éventrations.

On connaît également par le brevet US 4.769.038, une prothèse présentant trois couches de tissus, constituant deux feuillets ouverts raccordés à un feuillet unique. Cette prothèse a donc une structure générale en forme de Y, ce qui la rend difficilement applicable dans le cas d'une éventration médiane.

Les problèmes que se propose de résoudre l'invention sont de renforcer les tissus au niveau desquels est implantée la prothèse, d'améliorer son intégration dans les tissus et enfin d'augmenter sa solidité et son indéformabilité, tout en ayant une très grande souplesse, en ayant pour objectif essentiel, de traiter notamment, les différents types d'hernies et les éventrations.

Ainsi, la présente invention concerne généralement un élément prothétique en matériau textile destiné au traitement des éventrations comprenant deux couches parallèles et accouplées entre elles dans leur partie centrale en définissant ainsi une zone centrale tridimensionnelle et deux parties d'extrémité libres débordant de part et d'autre de ladite zone centrale, et aptes à enserrer partiellement un muscle, caractérisé en ce que ladite zone centrale tridimensionnelle est réalisée par tricotage au moyen d'un métier double fonture.

Pour résoudre les différents problèmes mentionnés ci-dessus, il a été conçu et mis au point un élément prothétique en matériau textile qui comprend deux couches parallèles de tissus accouplées d'une manière non jointive par un tricotage approprié pour créer au moins une zone plus épaisse de structure tridimensionnelle, l'une des couches au moins débordant de ladite zone.

Pour résoudre le problème posé de soigner une éventration, les deux couches débordent de part et d'autre de la zone tridimensionnelle, les parties débordantes étant libres. Les deux couches débordent latéralement de la zone centrale tridimensionnelle pour créer deux parties libres aptes à enserrer partiellement un muscle.

Avantageusement, les parties libres des couches de tissu coopèrent avec des agrafes.

Dans une forme de réalisation, notamment dans la cas où les différents éléments constitutifs sont de forme circulaire, la zone tridimensionnelle constituant une zone centrale, tandis que la couche débordante est disposée d'une manière concentrique, les agrafes sont fixées au pourtour de la zone centrale tridimensionnelle par un fil formant anneau, lesdites agrafes étant aptes à coopérer directement avec les muscles ou les aponévroses et sont recouvertes par les parties débordantes simples.

Pour résoudre le problème posé d'assurer une meilleure intégration de l'élément prothétique dans les tissus, chaque couche est composée de fibres polyester tricotées, lesdites couches étant reliées par des fils de même nature, l'ensemble étant imprégné de collagène purifié.

Le collagène est sélectionné pour favoriser la pénétration des cellules conjonctives pour une meilleure intégration.

L'invention est exposée, ci-après plus en détail à l'aide des dessins annexés, dans lesquels :
- La figure 1 est une vue en perspective de l'élément prothétique selon un mode de réalisation.
- La figure 2 est une vue en coupe longitudinale considérée selon la ligne 2.2 de la figure 1.
- La figure 3 est une vue en coupe montrant une éventration médiane.
- La figure 4 est une vue montrant un exemple d'application de l'élément prothétique conforme au mode de réalisation illustré aux figures 1 et 2 dans le cas d'une éventration médiane, montrée figure 3.

L'élément prothétique désigné dans son ensemble par (E), comprend deux couches parallèles de tissu (1) et (2) accouplées d'une manière non jointive, par un tricotage approprié (3), de manière à créer au moins une zone plus épaisse de structure tridimensionnelle. Cette structure tridimensionnelle est réalisée par un tissage double fonture.

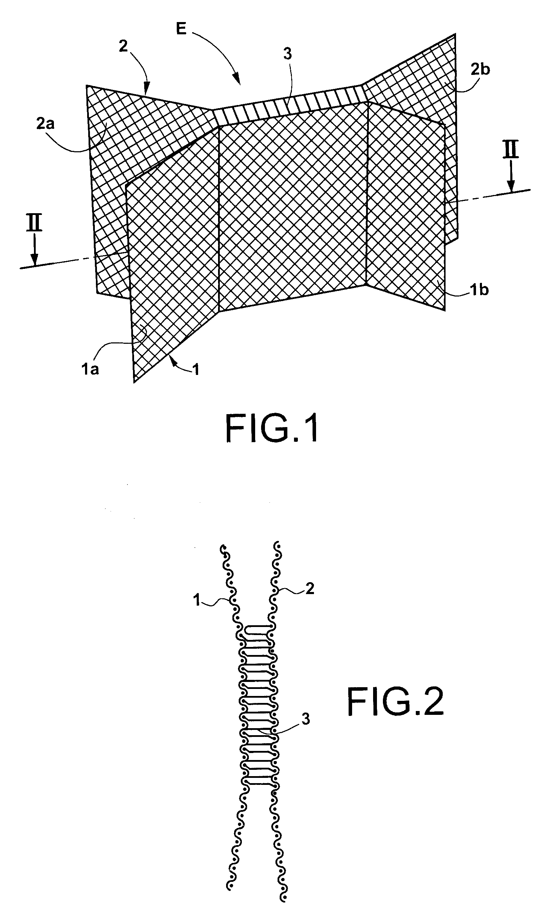

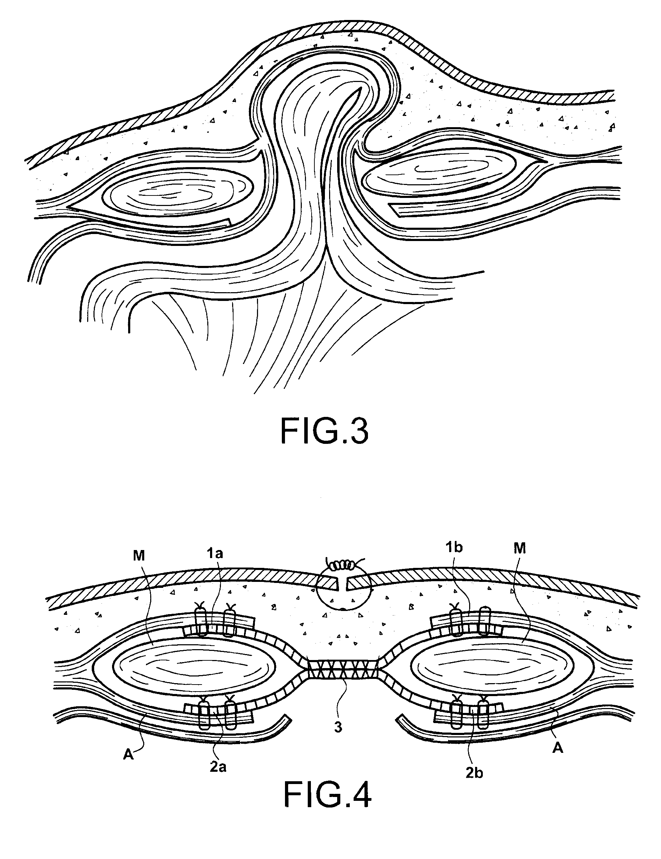

Dans le mode d'exécution illustré figures 1 et 2, les deux couches (1) et (2) débordent de part et d'autre de la zone centrale tridimensionnelle (3). Plus particulièrement, lesdites couches (1) et (2) débordent latéralement de la zone (3), pour constituer deux parties d'extrémité libres (1) (2a) et (1) (2b). Ainsi réalisé, l'élément prothétique trouve une application particulièrement avantageuse dans le cas d'éventration (figure 3).

La zone centrale (3) est disposée entre les muscles (M), tandis que les parties d'extrémité libres (1a) (2a) et (1b) (2b) recouvrent partiellement les muscles en y étant fixées par des agrafes ou autres, en combinaison avec les aponévroses (A) (figure 4).

Quelles que soient les formes de réalisations, chaque couche (1) et (2) est composée de fibres polyester tricotées reliée par des fils de même nature pour constituer la zone centrale tridimensionnelle (3). L'ensemble est imprégné de collagène purifié. Le collagène est sélectionné pour faciliter la pénétration des cellules conjonctives pour une meilleure intégration de l'élément prothétique.

A titre indicatif, le collagène est de type I purifié.

L'ensemble de l'élément prothétique, tel que défini est stérilisé notamment par rayonnement gamma.

Parmi les applications chirurgicales de l'élément prothétique, on peut citer :
- les hernies inguinales et crurales par voie trans-pariétale habituelle ou par coelioscopie,
- les éventrations,
- les prolapsus recto et génito-urinaires.

Bien évidemment, l'élément prothétique peut être réalisé en différentes dimensions.

Les avantages ressortent bien de la description, en particulier on souligne et on rappelle :
- le tricotage tridimensionnel confère à l'élément prothétique, une solidité accrue en le rendant indéformable, tout en gardant une grande souplesse,
- l'imprégnation des fibres par le collagène favorise leur colonisation par les cellules et permet une meilleure intégration de la prothèse dans les tissus.

## Revendications

1. Elément prothétique en matériau textile destiné au traitement des éventrations comprenant deux couches (1, 2) parallèles et accouplées entre elles dans leur partie centrale en définissant ainsi une zone centrale tridimensionnelle (3) et deux parties d'extrémité libres (1a, 2a; 1b, 2b) débordant de part et d'autre de ladite zone centrale (3), et aptes à enserrer partiellement un muscle, caractérisé en ce que ladite zone centrale tridimensionnelle (3) est réalisée par tricotage au moyen d'un métier double fonture.

2. Elément prothétique selon la revendication 1, caractérisé en ce que le tricotage est réalisé en fibres de polyester.

3. Elément prothétique selon la revendication 1 ou 2, caractérisé en ce que, après le tricotage, l'ensemble obtenu est imprégné de collagène purifié.

## Claims

1. A prosthetic element made of textile material, intended for treating ruptures, comprising two layers (1,2) which are parallel and are coupled together in their central part in thus defining a three-dimensional central zone (3) and two free end parts (1a, 2a; 1b, 2b) which extend at both sides from said central zone (3), and which are able to partially embrace a muscle, characterised in that said three-dimensional central zone (3) is made by knitting by means of a double needle bed loom.

2. The prosthetic element according to claim 1, characterised in that the knitting is made in polyester fibres.

3. The prosthetic element according to claim 1 or 2, characterised in that, after the knitting, the article obtained is impregnated with purified collagen.

## Patentansprüche

1. Prothetisches Element aus textilem Material, das zur Behandlung von Eventrationen vorgesehen ist und zwei parallele Schichten (1, 2) umfasst, die in ihrem Mittelteil miteinander verbunden sind, wobei sie einen dreidimensionalen mittleren Bereich (3) und zwei freie Endteile (1a, 2a; 1b, 2b) bilden, die auf beiden Seiten über den mittleren Bereich (3) hinausragen und in der Lage sind, einen Muskel teilweise zu erfassen, **dadurch gekennzeichnet, dass** dieser dreidimensionale mittlere Bereich (3) mittels einer Doppelkopf stückwirkmaschine durch Wirken hergestellt ist.

2. Prothetisches Element nach Anspruch 1, **dadurch gekennzeichnet, dass** der Wirkvorgang mit Polyesterfasern durchgeführt wird.

3. Prothetisches Element nach Anspruch 1 oder 2, **dadurch gekennzeichnet**, **dass** die nach dem Wirkvorgang erhaltene Einheit mit gereinigtem Kollagen imprägniert worden ist.
